# FASCICULE DE BREVET EUROPEEN

(11) **EP 1 694 668 B1**
(45) Date de publication et mention de la délivrance du brevet: **28.07.2010**
(21) Numéro de dépôt: 04805590.9
(22) Date de dépôt: 30.11.2004
(51) Int. Cl.: C07D 401/14, C07D 417/14

(54) **DERIVES DE 4-[(ARYLMETHYL)AMINOMETHYL]PIPERIDINE, LEUR PREPARATION ET LEUR APPLICATION EN THERAPEUTIQUE**
4-[(ARYLMETHYL)AMINOMETHYL]PIPERIDINDERIVATE, DEREN HERSTELLUNG UND DEREN ANWENDUNG IN THERAPEUTIKA
4-[(ARYLMETHYL)AMINOMETHYL]PIPERIDINE DERIVATIVES, PREPARATION THEREOF AND APPLICATION OF SAME IN THERAPEUTICS

(30) Priorité: 01.12.2003 FR 0314172
(43) Date de publication de la demande: 30.08.2006
(73) Titulaire: Sanofi-Aventis, 75013 Paris (FR)
(72) Inventeur: BOSCH, Michaël, F-34590 Marsillargues (FR); WAGNON, Jean, F-34070 Montpellier (FR)
(74) Mandataire: Kugel, Dominique
(86) Numéro de dépôt international: PCT/FR2004/003066
(87) Numéro de publication internationale: WO 2005/054229

(56) Documents cités:
- WO-A-00/69828
- WO-A-00/69829
- WO-A-01/49684
- WO-A-03/104225
- WO-A-03/104226

## Description

La présente invention a pour objet des dérivés de 4-[(arylméthyl)aminométhyl] pipéridine substitués, leur préparation et leur application en thérapeutique.

Les composés selon la présente invention présentent une affinité pour le récepteur p75^{NTR} des neurotrophines.

Les neurotrophines appartiennent à une famille de protéines possédant une structure et des fonctions proches et incluant le facteur de croissance nerveuse (NGF, de l'anglais Nerve Growth Factor), le BDNF, (de l'anglais Brain Derived Neurotrophic Factor), la neurotrophine-3 (NT-3, de l'anglais Neurotrophin-3), la neurotrophine-4/5 (NT-4/5, de l'anglais Neurotrophin-4/5) et la neurotrophine 6 (NT-6, de l'anglais Neurotrophin-6). Les effets biologiques de ces protéines (survie et différenciation) s'exercent par interaction avec des récepteurs membranaires à activité tyrosine kinase (trk-A, trk-B et trk-C) (H. THOENEN, Science, 1995, 270, 593-598 ; G.R. LEWIN et Y.A. BARDE, Annu. Rev. Neurosci., 1996, 19, 289-317 ; M.V. CHAO, J. Neurobiol., 1994, 25, 1373-1385 ; M. BOTHWELL, Annu. Rev. Neurosci., 1995, 18, 223-253 ; G. DECHANT et Y.A. BARDE, Curr. Opin. Neurobiol., 1997, 7, 413-418). Toutefois de nombreux travaux montrent le rôle prépondérant du récepteur p75^{NTR} dans l'activité des neurotrophines.

Le récepteur p75^{NTR}, récepteur de toutes les neurotrophines, est une glycoprotéine transmembranaire de la famille du récepteur du facteur de nécrose tumorale (TNF, de l'anglais Tumor Necrosis Factor) (W.J. FRIEDMAN et L.A. GREENE, Exp. Cell. Res., 1999, 253, 131-142 ; J. MELDOSIS et al., Trends Pharmacol. Sci., 2000, 21, 242-243). Plusieurs fonctions biologiques sont attribués au récepteur p75^{NTR} : d'une part, la modulation de l'affinité des neurotrophines pour les récepteurs trk ; d'autre part, en l'absence de trk, une induction d'un signal de mort cellulaire par apoptose qui s'effectue par homodimérisation du récepteur et activation de la voie des céramides.

L'apoptose ou mort cellulaire programmée est un mécanisme physiologique d'élimination des cellules dans de nombreux tissus. En particulier, l'apoptose joue un rôle prépondérant dans l'embryogénèse, la morphogénèse et le renouvellement cellulaire. L'apoptose est un phénomène génétiquement contrôlé qui n'intervient qu'à un stade avancé et irréversible de lésion cellulaire.

De nombreux travaux montrent que l'apoptose intervient dans plusieurs pathologies du système nerveux central comme la sclérose latérale amyotrophique, la sclérose en plaques, les maladies d'Alzheimer, de Parkinson et d' Huntington et les maladies à prion. De plus, la mort neuronale par apoptose intervient également très précocement après une ischémie cérébrale et cardiaque. La mort cellulaire est également un phénomène prépondérant dans l'athérosclérose, en effet on évalue à 80% les zones de nécrose dans les lésions primaires d'athérosclérose chez l'homme (M.L. BOCHATON-PIALAT et al., Am. J. Pathol., 1995, 146, 1-6 ; H. PERLMAN, Circulation, 1997, 95, 981-987). L'apoptose est également impliquée dans les mécanismes conduisant à la mort cellulaire consécutive à une ischémie-reperfusion cardiaque (H. YAOITA et al., Cardiovasc. Res., 2000, 45, 630-641).

Plusieurs travaux montrent que le signal pro-apoptotique p75^{NTR} dépendant est observé dans différents types cellulaires dont les cellules neuronales, les oligodendrocytes, les cellules de Schwann et aussi les cellules hépatiques, cardiaques et musculaires lisses (J.M. FRADE et al., Nature, 1996, 383, 166-168 ; P. LASACCIA-BONNEFIL et al., Nature, 1996, 383, 716-719 ; M. SOILU-HANNINEN et al., J. Neurosci., 1999, 19, 4828-4838 ; N. TRIM et al., Am. J. Pathol., 2000, 156, 1235-1243 ; S.Y. WANG et al., Am. J. Pathol., 2000, 157, 1247-1258). De plus, plusieurs expériences réalisées in vivo montrent une augmentation de l'expression de p75^{NTR} après ischémie dans des régions du cerveau et du coeur dans lesquelles une apoptose massive est enregistrée. Ces résultats suggèrent donc que p75^{NTR} peut jouer un rôle prépondérant dans les mécanismes conduisant à la mort neuronale par apoptose post ischémie (P.P. ROUX et al., J. Neurosci., 1999, 19, 6887-6896 ; J.A. PARK et al., J. Neurosci., 2000, 20 9096-9103).

Le récepteur p75^{NTR} est décrit comme cible cellulaire du peptide Prion (V. DELLA-BIANCA et al., J. Biol. Chem., 2001, in Press) et du peptide β-Amyloide (S. RABIZADEH et al., Proc. Natl. Acad. Sci. USA, 1994, 91, 10703-10706) et serait ainsi impliqué dans les phénomènes d'apoptose induits par ces composés. Ces résultats supportent l'hypothèse selon laquelle p75^{NTR} jouerait un rôle important dans la mort neuronale induite par la protéine prion infectieuse (encéphalopathie spongiforme transmissible) ou par la protéine beta Amyloide (maladie d'Alzheimer).

Des études récentes suggèrent que le récepteur p75^{NTR} jouerait également un rôle important dans la régénération axonale via sa fonction de co-récepteur du récepteur Nogo (WONG et al., Nature Neurosci., 2002, 5, 1302-1308 ; Kerracher and Winton, Neuron, 2002, 36, 345-348). En effet, plusieurs protéines associées à la myéline (myelin-associated glycoprotein, MAG, Nogo-A et oligodendrocyte myelin glycoprotein OMgp) inhibent la régénération nerveuse au niveau central lors d'un traumatisme médullaire ou crânien. Ces protéines sont localisées dans la membrane des oligodendrocytes directement adjacents à l'axone et inhibent la croissance neuritique en se liant avec une forte affinité au récepteur Nogo localisé sur la membrane axonale. Le récepteur p75^{NTR} est associé au récepteur Nogo et impliqué dans la signalisation des effects inhibiteurs de ces protéines de la myéline vis-à-vis de la croissance axonale. De ce fait, le récepteur p75^{NTR} joue un rôle majeur dans la régulation de la plasticité neuronale et dans les intéractions neurones-glie et représente ainsi une cible thérapeutique de choix pour promouvoir la régénération nerveuse.

Au niveau périphérique, des travaux récents montrent une augmentation de l'expression de p75^{NTR} et des neurotrophines et une apoptose massive dans des lésions d'athérosclérose. De plus, un effet pro-angiogène et vasodilateur du NGF est également enregistré. Enfin, une nouvelle forme de p75^{NTR} tronquée dans la partie extracellulaire a été mise en évidence ainsi que son rôle majeur dans la vasculogénèse établie (D. VON SHACK et al., Nature Neuroscience, 2001, 4, 977-978). L'ensemble de ces données récentes suggèrent que p75^{NTR} sous forme entière ou tronquée pourrait également jouer un rôle prépondérant dans les pathologies vasculaires.

Un certain nombre de composés sont connus pour interagir avec le système trkA/NGF/p75^{NTR} ou pour posséder une activité de type NGF. Ainsi la demande de brevet WO 00/59893 décrit des dérivés de pyrimidines substituées qui démontrent une activité de type NGF et/ou qui augmentent l'activité du NGF sur les cellules PC12. Les demandes de brevet WO 00/69828 et WO 00/69829 décrivent des composés polycycliques qui inhibent la liaison du NGF au récepteur p75^{NTR} dans des cellules qui n'expriment pas le récepteur trkA. La demande WO 94/11373 décrit des dérivés de pyridazinoquinazolone qui se lient au récepteur p75^{NTR} des neurotrophines. La demande WO 94/22866 décrit des dérivés de pyrazoloquinazolone qui se lient au NGF de manière spécifique afin d'éviter sa fixation au récepteur p75^{NTR} mais lui permettant d'interagir avec le récepteur trk. La demande WO 01/49684 décrit des dérivés de tétrahydropyridines substitués qui possèdent une activité vis-à-vis de la modulation du TNF-alpha.

La demande WO03/104225 décrit des dérivés de pipérazinylacylpipéridine qui présentent une affinité pour le récepteur p75^{NTR} des neurotrophines. La demande WO 03/104226 décrit des dérivés de pipérazinylarylpipéridine qui présentent une affinité pour le récepteur p75^{NTR} des neurotrophines.

On a maintenant trouvé de nouveaux dérivés de 4-[(arylméthyl)aminométhyl] pipéridine qui présentent une affinité pour le récepteur p73^{NTR}.

La présente invention a pour objet des composés répondant à la formule (I) : 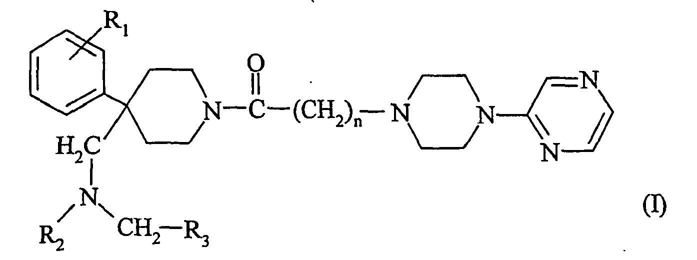 dans laquelle :
- n est 1 ou 2 ;
- R₁ représente un radical trifluorométhyle ;
- R₂ représente un atome d'hydrogène ou un (C₁-C₃)alkyle ;
- R₃ représente un groupe aromatique choisi parmi : 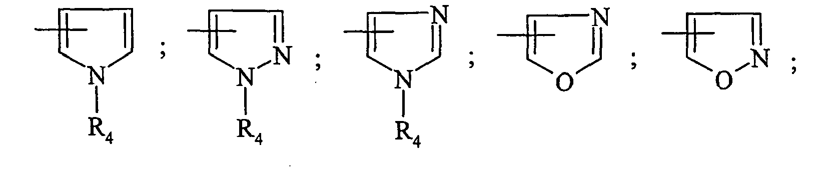 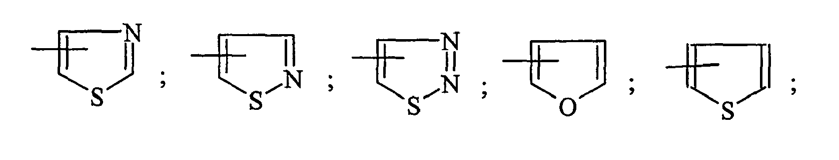 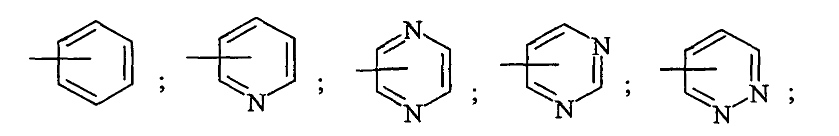 lesdits groupes aromatiques étant non substitués, mono- ou disubstitués par un substituant choisi indépendamment parmi un atome d'halogène, un (C₁-C₃)alkyle ou un (C₁-C₃)alcoxy ;
- R₄ représente un atome d'hydrogène ou un (C₁-C₃)alkyle.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables mais les sels d'autres acides utiles pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Par atome d'halogène on entend un atome de brome, de chlore, de fluor ou d'iode.

Par (C₁-C₃)alkyle on entend un radical alkyle linéaire ou ramifié de un à trois atomes de carbone tel que le radical méthyle, éthyle, propyle, isopropyle.

Par (C₁-C₃)alcoxy on entend un radical alcoxy linéaire ou ramifié de un à trois atomes de carbone, tel que le radical méthoxy, éthoxy, propoxy, isopropoxy.

Parmi les composés de formule (I) objets de l'invention, on peut citer les composés préférés qui se définissent comme suit :
- n est 1 ;
- et/ou R₁ est en position -3- du phényle et représente un radical trifluorométhyle ;
- et/ou R₂ représente un atome d'hydrogène ou un radical méthyle ;
- et/ou R₃ représente un radical 1-méthyl-1*H-*pyrrol-2-yle ; 1-méthyl-1*H-*imidazol-2-yle ; 1,3-thiazol-2-yle ; 2-furyle ; 3-furyle ; 5-méthyl-2-furyle ; 4,5-diméthyl-2-furyle ; 5-chloro-2-furyle ; 2-thiényle ; 3-thiényle ; phényle ; pyridin-2-yle ; pyridin-3-yle ; pyridin-4-yle ; pyrazin-2-yle ; 6-méthylpyridin-2-yle ; 3-méthyl-2-thiényle ; 5-méthyl-2-thiényle ; pyrimidin-5-yle ; 1*H-*imidazol-2-yle ; 1*H-*imidazol-5-yle ; 4-méthyl-1*H*-imidazol-5-yle.

Parmi ces derniers composés préférés, on préfère particulièrement les composés de formule (I) pour lesquels :
- n est 1 ;
- R₁ est en position -3- du phényle et représente un radical trifluorométhyle ;
- R₂ représente un atome d'hydrogène ou un radical méthyle ;
- R₃ représente un radical 1-méthyl-1*H-*pyrrol-2-yle ; 1-méthyl-1*H-*imidazol-2-yle ; 1,3-thiazol-2-yle ; 2-furyle ; 3-furyle ; 5-méthyl-2-furyle ; 4,5-diméthyl-2-furyle ; 5-chloro-2-furyle ; 2-thiényle ; 3-thiényle ; phényle ; pyridin-2-yle ; pyridin-3-yle ; pyridin-4-yle ; pyrazin-2-yle ; 6-méthylpyridin-2-yle ; 3-méthyl-2-thiényle ; 5-méthyl-2-thiényle ; pyrimidin-5-yle ; 1*H-*imidazol-2-yle ; 1*H-*imidazol-5-yle ; 4-méthyl-1*H-*imidazol-5-yle.
à l'état de base ou de sel d'addition à un acide, ainsi qu'à l'état d'hydrate ou de solvat.

Parmi les composés de formule (I) objets de l'invention, on peut notamment citer les composés suivants :
- [(1-méthyl-1*H-*pyrrol-2-yl)méthyl][[1-[(4-pyrazin-2-ylpipérazin-1-yl)acétyl]-4-[3-(trifluorométhyl)phényl]pipéridin-4-yl]méthyl]amine ;
- N-méthyl-1-(1-méthyl-1*H-*imidazol-2-yl)-N-[[1-[(4-pyrazin-2-ylpipérazin-1-yl)acétyl]-4-[3-(trifluorométhyl)phényl]pipéridin-4-yl]méthyl]méthanamine ;
- N-méthyl-1-[1-[(4-pyrazin-2-ylpipérazin-1-yl)acétyl]-4-[3-(trifluorométhyl) phényl]pipéridin-4-yl]-N-(1,3-thiazol-2-ylméthyl)méthanamine ;
- (2-furylméthyl)[[1-[(4-pyrazin-2-ylpipérazin-1-yl)acétyl]-4-[3-(trifluorométhyl) phényl]pipéridin-4-yl]méthyl]amine ;
- (3-furylméthyl)[[1-[(4-pyrazin-2-ylpipérazin-1-yl)acétyl]-4-[3-(trifluorométhyl) phényl]pipéridin-4-yl]méthyl]amine ;
- [(5-méthyl-2-furyl)méthyl][[1-[(4-pyrazin-2-ylpipérazin-1-yl)acétyl]-4-[3-(trifluorométhyl)phényl]pipéridin-4-yl]méthyl]amine ;
- [(4,5-diméthyl-2-furyl)méthyl]méthyl[[1-[(4-pyrazin-2-ylpipérazin-1-yl)acétyl]-4-[3-(trifluorométhyl)phényl]pipéridin-4-yl]méthyl]amine ;
- [(5-chloro-2-furyl)méthyl]méthyl[[1-[(4-pyrazin-2-ylpipérazin-1-yl)acétyl]-4-[3-(trifluorométhyl)phényl]pipéridin-4-yl]méthyl]amine ;
- [[1-[(4-pyrazin-2-ylpipérazin-1-yl)acétyl]-4-[3-(trifluorométhyl)phényl]pipéridin-4-yl]méthyl](2-thiénylméthyl)amine ;
- [[1-[(4-pyrazin-2-ylpipérazin-1-yl)acétyl]-4-[3-(trifluorométhyl)phényl]pipéridin-4-yl]méthyl](3-thiénylméthyl)amine ;
- 1-phényl-N-[[1-[(4-pyrazin-2-ylpipérazin-1-yl)acétyl]-4-[3-(trifluorométhyl) phényl]pipéridin-4-yl]méthyl]méthanamine ;
- [[1-[(4-pyrazin-2-ylpipérazin-1-yl)acétyl]-4-[3-(trifluorométhyl)phényl]pipéridin-4-yl]méthyl](pyridin-2-ylméthyl)amine ;
- N-méthyl-1-[1-[(4-pyrazin-2-ylpipérazin-1-yl)acétyl]-4-[3-(trifluorométyl) phényl]pipéridin-4-yl]-N-(pyridin-2-ylméthyl)méthanamine ;
- N-méthyl-1-[1-[(4-pyrazin-2-ylpipérazin-1-yl)acétyl]-4-[3-(trifluorométhyl) phényl]pipéridin-4-yl]-N-(pyridin-3-ylméthyl)méthanamine ;
- N-méthyl-1-[1-[(4-pyrazin-2-ylpipérazin-1-yl)acétyl]-4-[3-(trifluorométhyl) phényl]pipéridin-4-yl]-N-(pyridin-4-ylméthyl)méthanamine ;
- N-méthyl-1-pyrazin-2-yl-N-[[1-[(4-pyrazin-2-ylpipérazin-1-yl)acétyl]-4-[3-(trifluorométhyl)phényl]pipéridin-4-yl]méthyl]méthanamine ;
- [(6-méthylpyridin-2-yl)méthyl][[1-[(4-pyrazin-2-ylpipérazin-1-yl)acétyl]-4-[3-(trifluorométhyl)phényl]pipéridin-4-yl]méthyl]amine ;
- [(3-méthyl-2-thiényl)méthyl][[1-[(4-pyrazin-2-ylpipérazin-1-yl)acétyl]-4-[3-(trifluorométhyl)phényl]pipéridin-4-yl]méthyl]amine ;
- N-méthyl-1-(5-méthyl-2-thiényl)-N-[1-[(4-pyrazin-2-ylpipérazin-1-yl)acétyl]-4-[3-(trifluorométhyl)phényl]pipéridin-4-yl]méthyl]méthanamine ;
- N-méthyl-1-[1-[(4-pyrazin-2-ylpipérazin-1-yl)acétyl]-4-[3-(trifluorométhyl) phényl]pipéridin-4-yl]-N-(pyrimidin-5-ylméthyl]méthanamine ;
- (1*H-*imidazol-2-ylméthyl)méthyl[[1-[(4-pyrazin-2-ylpipérazin-1-yl)acétyl]-4-[3-(trifluorométhyl)phényl]pipéridin-4-yl]méthyl]amine ;
- (1*H-*imidazol-5-ylméthyl)méthyl[[1-[(4-pyrazin-2-ylpipérazin-1-yl)acétyl]-4-[3-(trifluorométhyl)phényl]pipéridin-4-yl]méthyl]amine ;
- N-méthyl-1-(4-méthyl-1H-imidazol-5-yl)-N-[[1-[(4-pyrazin-2-ylpipérazin-1-yl)acétyl]-4-[3-(trifluorométhyl)phényl]pipéridin-4-yl]méthyl]méthanamine;
à l'état de base ou de sel d'addition à une acide, ainsi qu'à l'état d'hydrate ou de solvat.

Dans ce qui suit, on entend par groupe protecteur Pg un groupe qui permet, d'une part, de protéger une fonction réactive telle qu'une amine pendant une synthèse et, d'autre part, de régénérer la fonction réactive intacte en fin de synthèse. Des exemples de groupes protecteurs ainsi que des méthodes de protection et de déprotection sont données dans "Protective Groupe in Organic Synthesis", Green et al., 2nd Edition (John Wiley & Sons, Inc., New York).

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans "Advances in Organic Chemistry", J. March, 3rd Edition, Wiley Interscience, p. 310-316.

Conformément à l'invention, on peut préparer les composés de formule (I) selon un procédé caractérisé en ce que :
on fait réagir un composé de formule : 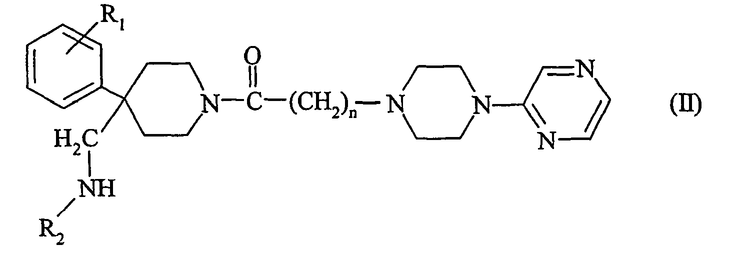 dans laquelle n, R₁ et R₂ sont tels que définis pour un composé de formule (I), avec un composé de formule : 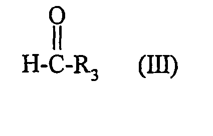 dans laquelle R₃ est tel que défini pour un composé de formule (I), en présence d'un acide, dans un solvant, puis on réduit le sel d'iminium formé intermédiairement au moyen d'un agent réducteur.

Eventuellement, on transforme le composé de formule (I) en l'un de sels d'addition à un acide.

La réaction s'effectue en présence d'un acide tel que l'acide acétique, dans un solvant tel que le dichlorométhane ou le tétrahydrofurane, à une température comprise entre la température ambiante et la température de reflux du solvant, et forme *in-situ* une imine intermédiaire qui est réduite chimiquement en utilisant, par exemple, le cyanoborohydrure de sodium ou le triacétoxyborohydrure de sodium ou catalytiquement en utilisant l'hydrogène et un catalyseur tel que le palladium sur charbon ou le nickel de Raney^{®}.

Les composés de formule (I) ainsi obtenus peuvent être ultérieurement séparés du milieu réactionnel et purifiés selon les méthodes classiques, par exemple par cristallisation ou chromatographie.

Les composés de formule (I) ainsi obtenus sont isolés sous forme de base libre ou de sel, selon les techniques classiques.

Les composés de formule (II) se préparent par réaction de déprotection d'un composé de formule : 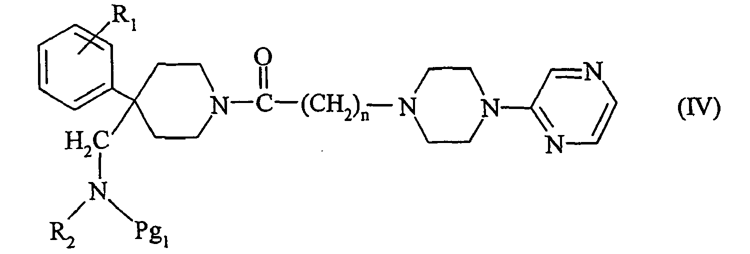 dans laquelle n, R₁ et R₂ sont tels que définis pour un composé de formule (I) et Pg₁ représente un groupe N-protecteur classique tel qu'un groupe *tert*-butoxycarbonyle, un groupe benzyloxycarbonyle ou un groupe benzyle. La réaction de déprotection s'effectue selon les méthodes précédemment citées.

Les composés de formule (III) sont disponibles dans le commerce ou décrits dans la littérature, ou bien peuvent être préparés selon des méthodes qui y sont décrites telles que dans J. Org. Chem., 1961, 26, 2976 ; Chim. Ind. (Milan), 1968, 50, 264 ; J. Med. Chem., 1970, 13, 1208-1212 ; J. Heterocycl. Chem., 1990, 27 (1), 1-12 ; Synth. Commun., 1995, 25 (9), 1383-1390 ; Bioorg. Med. Chem. Lett., 2003, 13 (3), 463-466.

Les composes de formule (IV) se préparent :
- par réaction d'un composé de formule : 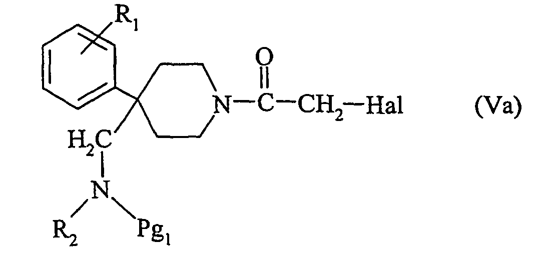 dans laquelle R₁, R₂ et Pg₁ sont tels que définis précédemment, et Hal représente un atome d'halogène, de préférence le chlore ou le brome, lorsqu'on veut obtenir un composé de formule (IV) dans laquelle n = 1.
- ou par réaction d'un composé de formule : 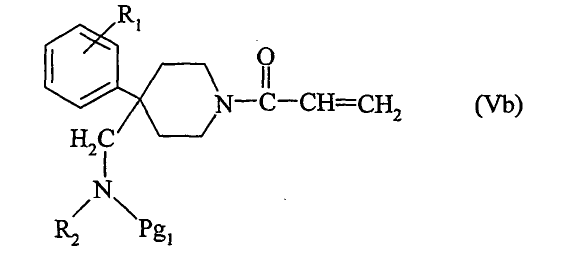 dans laquelle R₁, R₂ et Pg₁ sont tels que définis précédemment, lorsqu'on veut obtenir un composé de formule (IV) dans laquelle n = 2 ;
avec la 1-(2-pyrazinyl)pipérazine.

Lorsqu'on fait réagir un composé de formule (Va) ou (Vb) avec la 1-(2-pyrazinyl)pipérazine, la réaction s'effectue en présence d'une base choisie parmi les bases organiques telles que la triéthylamine, la N,N-diisopropyléthylamine ou la N-méthylmorpholine ou parmi les carbonates ou bicarbonates de métal alcalin tels que le carbonate de potassium, le carbonate de sodium ou le bicarbonate de sodium et en l'absence ou en présence d'un iodure de métal alcalin tel que l'iodure de potassium ou l'iodure de sodium. La réaction s'effectue dans un solvant tel que l'acétonitrile, le N,N-diméthylformamide, le toluène ou le propan-2-ol et à une température comprise entre la température ambiante et la température de reflux du solvant.

Les composés de formule (Va) se préparent par réaction d'un dérivé de pipéridine de formule : 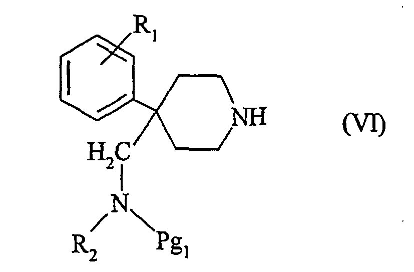 dans laquelle R₁, R₂ et Pg₁ sont tels que définis précédemment, avec un composé de formule : 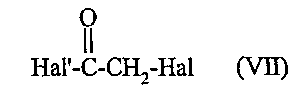 dans laquelle Hal et Hal' représentent chacun indépendamment un atome d'halogène, de préférence le chlore ou le brome. La réaction s'effectue en présence d'une base telle que la triéthylamine, la N,N-diisopropyléthylamine ou la N-méthylmorpholine, dans un solvant tel que le dichlorométhane, le tétrahydrorurane, le dioxane ou un mélange de ces solvants et à une température comprise entre 0°C et la température ambiante.

Les composés de formule (Vb) se préparent par réaction du composé de formule (VI) avec un composé de formule : 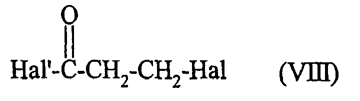 dans Hal et Hal' sont tels que définis ci-dessus, dans les conditions opératoires ci-dessus mentionnées.

Les composés de formule (VI) se préparent selon le SCHEMA 1 ci-après dans lequel R₁ et R₂ sont tels que définis pour un composé de formule (I), et Pg₁ et Pg₂ représentent des groupes N-protecteurs différents l'un de l'autre et choisis parmi les groupes N-protecteurs classiques bien connus de l'homme de l'art.

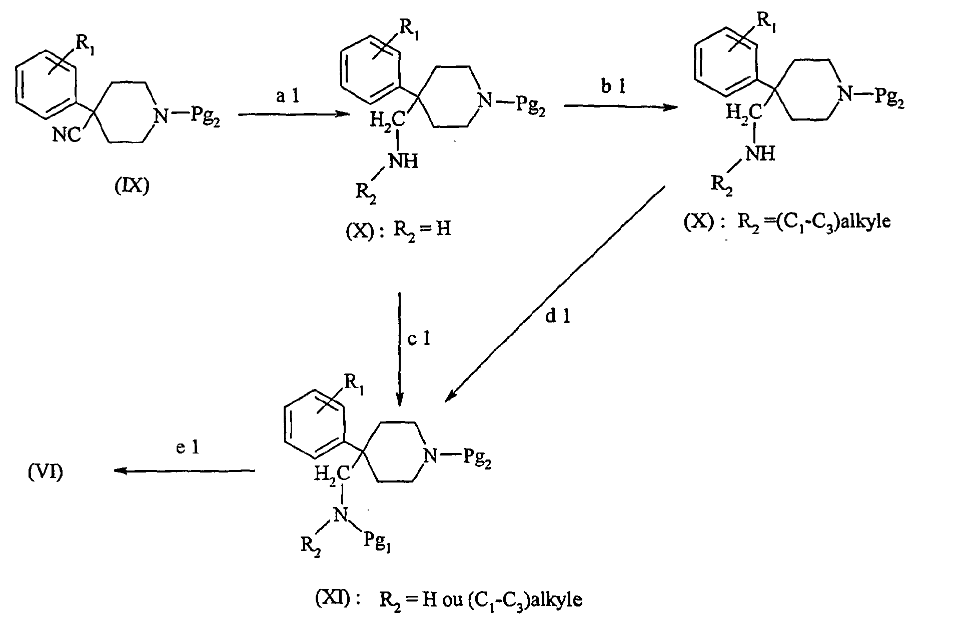

A l'étape a1, on réduit le groupe cyano d'un composé de formule (IX) pour obtenir un composé de formule (X) dans laquelle R₂ = H. La réduction s'effectue, par exemple, par hydrogénation en présence d'un catalyseur tel que le nickel de Raney^{®} ou le rhodium sur alumine, en présence ou en l'absence d'ammoniaque, dans un solvant tel que le méthanol, le N,N-diméthylformamide ou le tétrahydrofurane ou un mélange de ces solvants et à une température comprise entre la température ambiante et 60°C.

Un composé de formule (X) dans laquelle R₂ = (C₁-C₃)alkyle se prépare, à l'étape b1, par réaction d'alkylation classique ou par réaction d'acylation puis réduction à partir d'un composé de formule (X) dans laquelle R₂ = H. Ainsi, par exemple, lorsqu'on veut préparer un composé de formule (X) dans laquelle R₂ = -CH₃, on fait réagir un composé de formule (X) dans laquelle R₂ = H avec le formiate d'éthyle, à une température comprise entre la température ambiante et 60°C, puis réduit le composé intermédiaire correspondant dans lequel R₂ = -CHO au moyen d'un agent réducteur tel que l'hydrure d'aluminium et de lithium, dans un solvant tel que l'éther diéthylique ou le tétrahydrofurane et à une température comprise entre la température ambiante et la température de reflux du solvant.

A l'étape c1 ou à l'étape d1 on protège la fonction amine du composé de formule (X) par introduction du groupe N-protecteur Pg₁ différent de Pg₂ selon les méthodes classiques puis élimine, à l'étape e1, le groupe N-protecteur Pg₂ selon les méthodes classiques pour obtenir un composé de formule (VI) attendu.

Les composés de formule (VII) ou (VIII) seon commerciaux, connus ou se préparent selon des méthodes connues.

Les composés de formule (IX) se préparent selon des méthodes connues telles que celles décrites dans Bioorg. Med. Chem. Lett., 1999, 9, 3273-3276 ou dans J. Med. Chem., 1999, 42 (23), 4778-4793.

La 1-(2-pyrazinyl)pipérazine est commerciale ou se prépare par réaction de la pipérazine avec la 2-chloropyrazine en présence d'une base telle qu'un carbonate de métal alcalin comme le carbonate de potassium, dans un solvant tel que l'éthanol, le propan-2-ol ou le n-butanol et à une température comprise entre la température ambiante et la température de reflux du solvant.

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans le TABLEAU I ci-après, qui illustre les structures chimiques et les propriétés physiques de quelques composés selon l'invention.

Dans les Préparations et dans les Exemples on utilise les abréviations suivantes:
éther : éther diéthylique
éther iso : éther diisopropylique
DMSO : diméthylsulfoxyde
DMF : N,N-diméthylformamide
THF : tétrahydrofurane
DCM : dichlorométhane
AcOEt : acétate d'éthyle
DIPEA : diisopropyléthylamine
TFA : acide trifluoroacétique
Ether chlorhydrique 2N : solution 2N d'acide chlorhydrique dans l'éther diéthylique
F : point de fusion
TA : température ambiante
Eb : température d'ébullition
CLHP : chromatographie liquide haute performance
Silice H : gel de silice 60 H commercialisé par Merck (DARMSTAD)
Solution tampon pH = 2 : solution de 16,66 g de KHSO₄ et 32,32 g de K₂SO₄ dans 1 litre d'eau.

Les spectres de résonance magnétique du proton (RMN ¹H) sont enregistrés à 200 MHz dans du DMSO-d₆, en utilisant le pic du DMSO-d₆ comme référence. Les déplacements chimiques δ sont exprimés en parties par million (ppm). Les signaux observés sont exprimés ainsi : s: singulet ; se : singulet élargi ; d : doublet ; d.d : doublet dédoublé ; t : triplet ; td : triplet dédoublé ; q : quadruplet ; m : massif ; mt : multiplet.

Les spectres RMN confirment les structures des composés.

Les composés selon l'invention sont analysés par couplage LC/UV/MS (chromatographie liquide/détection UV/spectrométrie de masse).

Pour les composés on vérifie que leur spectre de masse obtenus en mode Electrospray positif (ESI+) sont compatibles avec la masse molaire calculée.

Les spectres de masse des composés selon l'invention présentent, en général, comme pic de base l'ion moléculaire MH⁺.

### PREPARATIONS

### 1. Préparations des composés de formule (VI).

### Préparation 1.1

### [4-[3-(Trifluorométhyl)phényl]-4-pipéridinyl]méthylcarbamate de tert-butyle.

(VI) : R₁= 3-CF₃ ; R₂ = H ; Pg₁ = -COOC(CH₃)₃.

### A) 2-(2,2-diéthoxyéthyl)-4,4-diéthoxy-2-[3-(trifluorométhyl)phényl]butanenitrile.

On laisse 5 minutes à TA sous agitation un mélange de 30 g de 3-(trifluorométhyl)phénylacétonitrile et 14,4 g d'amidure de sodium dans 400 ml de toluène, ajoute 66 ml de bromoacétaldéhyde diéthylacétal puis chauffe à 60°C pendant 3 heures. On concentre sous vide, reprend le résidu à l'eau, extrait à l'éther, sèche la phase organique sur Na₂SO₄ et évapore le solvant sous vide. On chromatographie le résidu sur gel de silice H en éluant par le mélange DCM/AcOEt (100/5 ; v/v). On obtient 26 g du produit attendu. B) 4-Oxo-2-(2-oxoéthyl)-2-[3-(trifluorométhyl)phényl]butanenitrile.

On laisse 1 heure sous agitation à 50°C un mélange de 23,9 g du composé obtenu à l'étape précédente dans 90 ml d'acide formique. On ajoute de l'eau au mélange réactionnel, extrait à l'AcOEt, lave la phase organique à l'eau, par une solution de NaHCO₃ à 10 %, sèche sur Na₂SO₄ et évapore le solvant sous vide. On obtient 16 g du produit attendu que l'on utilise immédiatement à l'étape suivante. C) Chlorhydrate de 1-benzyl-4-[3-(trifluorométhyl)phényl]-4-pipéridinecarbonitrile.

On laisse une nuit sous agitation à TA un mélange de 16 g du composé obtenu à l'étape précédente, 6,25 ml de benzylamine, 48,6 g de triacétoxyborohydrure de sodium et 5 gouttes d'acide acétique dans 150 ml de DCM. On ajoute ensuite, goutte à goutte, 40 ml de MeOH puis chauffe à 60°C pendant 1 heure. On concentre sous vide le mélange réactionnel, extrait le résidu à l'AcOEt, lave la phase organique par une solution de NaHCO₃ à 10 %, à l'eau, sèche sur Na₂SO₄ et évapore le solvant sous vide. On reprend le résidu dans une solution saturée d'HCl gaz dans l'éther et essore le précipité formé. On obtient 18 g du produit attendu. D) [1-Benzyl-4-[3-(trifluorométhyl)phényl]-4-pipéridinyl]méthylamine.

On hydrogène pendant une nuit, à TA et à pression atmosphérique, un mélange de 1,5 g du composé obtenu à l'étape C de la Préparation 1.4, 0,15 g de Nickel de Raney^{®} et 5 ml d'ammoniaque dans 20 ml de MeOH. On filtre le catalyseur et concentre sous vide le filtrat. On obtient 1,45 g du produit attendu. E) [1-Benzyl-4-[3-(trifluorométhyl)phényl]-4-pipéridinyl]méthylcarbamate de *tert-*butyle.

On chauffe à 40°C un mélange de 1,45 g du composé obtenu à l'étape précédente dans 20 ml d'AcOEt, ajoute 0,9 g de di-*tert*-butyldicarbonate puis chauffe à reflux pendant 30 minutes. Après refroidissement à TA, on ajoute de l'eau, extrait à l'AcOEt, sèche la phase organique sur Na₂SO₄ et évapore le solvant sous vide. On obtient 1,86 g du produit attendu. F) [4-[3-(trifluorométhyl)phényl]-4-pipéridinyl]méthylcarbamate de *tert*-butyle.

On hydrogène pendant une nuit, à TA et à pression atmosphérique, un mélange de 1,8 g du composé obtenu à l'étape précédente et 0,18 g de palladium sur charbon à 10 % dans 20 ml de MeOH. On filtre le catalyseur et concentre sous vide le filtrat. On obtient 1,3 g du produit attendu sous forme d'huile.

### Préparation 1.2

### [[4-[3-(Trifluorométhyl)phényl]pipéridin-4-yl]méthyl]carbamate de tert-butyle méthyle ;

(VI) : R₁ = 3-CF₃ ; R₂ = -CH₃ ; Pg₁ - -COOC(CH₃)₃

### A) N,N-bis-(2-chloroéthyl)benzylamine.

On refroidit au bain de glace un mélange de 150 g de chlorhydrate de N,N-bis-(2-chloroéthyl)amine et 100 ml de bromure de benzyle dans 1000ml de DMF, puis ajoute en goutte à goutte, 120ml de triéthylamine et laisse une nuit sous agitation à TA. On concentre sous vide le mélange réactionnel, reprend le résidu à l'eau, extrait 3 fois à l'éther, sèche les phases organiques sur Na₂SO₄ et évapore le solvant sous vide. On obtient 113 g du produit attendu. B) Chlorhydrate de 1-benzyl-4-[3-(trifluorométhyl)phényl]-4-pipéridinecarbonitrile.

A une suspension de 23,24 g d'hydrure de sodium à 60 % dans l'huile dans 100 ml de DMSO et 100 ml de THF, on ajoute, en goutte à goutte, sous atmosphère inerte et à TA, une solution de 50 g de 3-(trifluorométhyl)phénylacétonitrile dans 150 ml de DMSO et laisse 15 minutes sous agitation. On ajoute ensuite, en 1 heure, une solution de 62,43 g du composé obtenu à l'étape précédente dans 150ml de DMSO et laisse une nuit sous agitation à TA. On ajoute un mélange glace/eau, extrait à l'éther, sèche la phase organique sur Na₂SO₄ et évapore le solvant sous vide. On reprend le résidu dans 1000 ml d'EtOH chaud, laisse 48 heures sous agitation à TA et essore le produit cristallisé formé. On obtient 50 g du produit attendu. C) [1-Benzyl-4-[3-(trifluorométhyl)phényl]-4-pipéridinyl]méthylamine.

On dissout 30 g du composé obtenu à l'étape précédente dans une solution de NaOH à 10 %, extrait à l'éther, sèche la phase organique sur Na₂SO₄ et évapore le solvant sous vide. On reprend le produit sous forme de base libre dans 500 ml de MeOH et 30ml d'une solution d'ammoniaque à 20 %, on ajoute 3 g de nickel de Raney® et hydrogène pendant une nuit à TA et à pression atmosphérique. On filtre le catalyseur et concentre sous vide le filtrat. On reprend le résidu à l'eau, extrait à l'AcOEt, sèche la phase organique sur Na₂SO₄ et évapore le solvant sous-vide. On obtient 27 g du produit attendu. D) [[1-Benzyl-4-[3-(trifluorométhyl)phényl]-4-pipéridinyl]méthyl]formamide.

On laisse une nuit sous agitation à TA un mélange de 27 g du composé obtenu à l'étape précédente et 300 ml de formiate d'éthyle, puis chauffe à 60°C pendant 6 heures et laisse 48 heures sous agitation à TA. On concentre sous vide, reprend le résidu par une solution d'HCl à 10 %, lave la phase aqueuse acide à l'éther, ajoute de la glace et alcalinise par ajout d'une solution de NaOH à 10%, extrait à l'éther, sèche la phase organique sur Na₂SO₄ et évapore le solvant sous vide. On chromatographie le résidu sur gel de silice H en éluant au DCM puis par le mélange DCM/MeOH (100/4 ; v/v). On obtient 20 g du produit attendu. E) [[1-Benzyl-4-[3-(trifluorométhyl)phényl]-4-pipéridinyl]méthyl]méthylamine.

A une suspension de 4 g d'hydrure de lithium et d'aluminium dans 400 ml d'éther, on ajoute à TA 20 g du composé obtenu à l'étape précédente puis laisse 16 heures sous agitation à TA. On ajoute ensuite successivement 3 ml d'eau, 3 ml de NaOH à 30 % et 1 ml d'eau et laisse sous agitation. On filtre les sels minéraux sur Célite, décante le filtrat, sèche la phase organique sur Na₂SO₄ et évapore le solvant sous vide. On obtient 18 g du produit attendu. F) [[1-Benzyl-4-[3-(trifluorométhyl)phényl]-4-pipéridinyl]méthyl]méthylcarbamate de *tert*-butyle.

On laisse 1 heure sous agitation à TA un mélange de 18 g du composé obtenu à l'étape précédente et 9,6 g de di-*tert*-butyldicarbonate dans 300ml de DCM. On ajoute de l'eau au mélange réactionnel, extrait au DCM, sèche la phase organique sur Na₂SO₄ et évapore le solvant sous vide. On chromatographie le résidu sur gel de silice H en éluant par le mélange DCM/MeOH (100/2 ; v/v). On obtient 21g du produit attendu. G) [4-[3-(trifluorométhyl)phényl]pipéridin-4-yl]méthyl]carbamate de *tert*-butyle méthyle.

On hydrogène pendant 12 heures, à TA et à pression atmosphérique, un mélange de 21 g du composé obtenu à l'étape précédente et 2 g de palladium sur charbon à 10 % dans 300 ml de MeOH. On filtre le catalyseur et concentre sous vide le filtrat. On obtient 16 g du produit attendu.

### 2. Préparations des composés de formule (V).

### Préparation 2.1

### [1-(2-Chloroacétyl)-4-[3-(trifluorométhyl)phényl]-4-pipéridinyl]méthylcarbamate de tert-butyle.

(Va) : R₁ = 3-CF₃ ; R₂ = H ; Pg₁ = -COOC(CH₃)₃, ; Hal = Cl.

On refroidit au bain de glace un mélange de 4,95 g du composé obtenu à la Préparation 1.1 et 6,8 ml de triéthylamine dans 50 ml de DCM, ajoute, goutte à goutte, 1,65 ml de chlorure de 2-chloroacétyle et laisse sous agitation en laissant remonter la température à TA. On concentre sous vide, reprend le résidu par une solution saturée de K₂CO₃, extrait à l'AcOEt, lave la phase organique par une solution saturée de K₂CO₃, par une solution tampon pH = 2, par une solution saturée de NaCl, sèche sur Na₂SO₄ et évapore sous vide. On chromatographie le résidu sur gel de silice en éluant par le mélange DCM/AcOEt (80/20 ; v/v). On obtient 1,8 g du produit attendu que l'on utilise tel quel.

### Préparation 2.2

### [[1-(2-chloroacétyl)-4-[3-(trifluorométhyl)phényl]-4-pipéridinyl]méthyl]méthyl-carbamate de tert-butyle.

(Va) : R₁ = 3-CF₃ ; R₂ = -CH₃ ; Pg₁ = -COOC(CH₃)₃, ; Hal = Cl.

On refroidit à -40°C une solution de 14 g du composé obtenu à la Préparation 1.2 et 5,5 ml de triéthylamine dans 300 ml de DCM, ajoute lentement 3,1 ml de chlorure de 2-chloroacétyle et laisse sous agitation en laissant remonter la température à TA. On concentre sous vide, reprend le résidu à l'eau, extrait à l'AcOEt, lave la phase organique au tampon pH = 2, à l'eau, sèche sur Na₂SO₄ et évapore le solvant sous vide. On obtient 15,33 g du produit attendu.

### 3. Préparations des composés de formule (IV).

### Préparation 3.1

### [1-[2-[4-(2-pyrazinyl)-1-pipérazinyl]acétyl]-4-[3-(trifluorométhyl)phényl]-4-pipéridinyl]méthylcarbamate de tert-butyle.

(IV) : R₁ = 3-CF₃ ; R₂ = H ; Pg₁ = -COOC(CH₃)₃, ; n = 1.

On laisse 3 heures sous agitation à TA un mélange de 2,8 g du composé obtenu à la Préparation 2.1, 1,25 g de 1-(2-pyrazinyl)pipérazine, 1,1 g d'iodure de potassium et 1,8 g de K₂CO₃ dans 30 ml d'acétonitrile. On ajoute une solution saturée de K₂CO₃, extrait à l'AcOEt, sèche la phase organique sur Na₂SO₄ et évapore le solvant sous vide. On chromatographie le résidu sur gel de silice en éluant par le mélange DCM/MeOH (97/3 : v/v). On obtient 1,75 g du produit attendu.

### Préparation 3.2

### [[1-[2-[4-(2-pyrazinyl)-1-pipérazinyl]acétyl]-4-[3-(trifluorométhyl)phényl]-4-pipéridinyl]méthyl]carbamate de tert-butyle méthyle.

(IV) : R₁= 3-CF₃ ; R₂ = -CH₃ ; Pg₁ = -COOC(CH₃)₃, ; n = 1.

On laisse 5 heures sous agitation à TA un mélange de 10 g du composé obtenu à la Préparation 2.2, 3,7 g de 1-(2-pyrazinyl)pipérazine, 3,7 g d'iodure de potassium et 6,2 g de K₂CO₃ dans 200 ml d'acétonitrile. On concentre sous vide, reprend le résidu à l'eau, extrait à l'AcOEt, sèche la phase organique sur Na₂SO₄ et évapore le solvant sous vide. On chromatographie le résidu sur gel de silice H en éluant au DCM puis par le mélange DCM/MeOH (100/2 ; v/v). On obtient 10,7 g du produit attendu.

### 4. Préparations des composés de formule (II).

### Préparation 4.1 1-[4-(aminométhyl)-4-[3-(trifluorométhyl)phényl]-1-pipéridinyl]-2-[4-(2-pyrazinyl)-1-pipérazinyl]-1-éthanone.

(II): R₁=3-CF₃;R₂=H;n=1.

On laisse 4 heures sous agitation à TA un mélange de 1,7 g du composé obtenu à la Préparation 3.1, 50 ml d'une solution d'HCl 2N dans l'éther et 30 ml de MeOH. On concentre sous vide, reprend le résidu à l'eau, lave la phase aqueuse à l'AcOEt, alcalinise la phase aqueuse par ajout de K₂CO₃, extrait à l'AcOEt, lave la phase organique par une solution saturée de NaCl, sèche sur Na₂SO₄ et évapore le solvant sous vide. Le produit cristallisant à l'évaporation dans l'AcOEt, on essore les cristaux formés. On obtient 1,05 g du produit attendu, F =152-153°C.
Spectre de masse : MH⁺ = 463,3.

### Préparation 4.2

### 1-[4-[(Méthylamino)méthyl]-4-[3-(trifluorométhyl)phényl]-1-pipéridinyl]-2-[4-(2-pyrazinyl)-1-pipérazinyl]-1-éthanone.

(II) : R₁ = 3-CF₃ ; R₂ = -CH₃ ; n = 1.

A une solution de 8 g du composé obtenu à la Préparation 3.2 dans 100 ml de MeOH, on ajoute 300 ml d'une solution d'éther chlorhydrique 2N et laisse une nuit sous agitation à TA. On concentre sous vide, reprend le résidu par une solution de NaOH à 10 %, extrait à l'AcOEt, sèche la phase organique sur Na₂SO₄ et évapore le solvant sous vide. On chromatographie le résidu sur gel de silice H en éluant par le mélange DCM/MeOH (100/2 ; v/v) puis par le mélange DCM/MeOH/eau (100/5/0,5 ; v/v/v/). On obtient 4,5 g du produit attendu après cristallisation dans l'éther iso, F = 137-139°C.
Spectre de masse MH⁺ = 477,4.
RMN¹H : DMSO-d₆ : δ (ppm) : 1,10 : s : 1H ; 1,6-2,3 : m : 7H ; 2,4-3,8 : m : 16H ; 7,4-7,75 : m : 4H ; 7,8 : d : 1H ; 8,15 : dd : 1H ; 8,3 : d : 1H.

### EXEMPLE 1 : Composé n° 1

### [(1-Méthyl-1H-pyrrol-2-yl)méthyl][[1-[(4-pyrazin-2-ylpipérazin-1-yl)acétyl]-4-[3-(trifluorométhyl)phényl]pipéridin-4-yl]méthyl]amine.

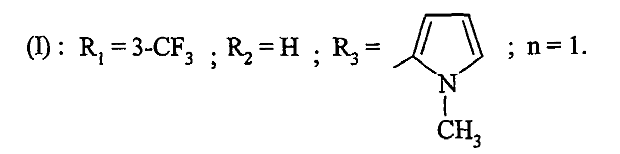

A un mélange de 1 g du composé obtenu à la Préparation 4.1, 0,235 ml de 1-méthyl-2-pyrrolecarboxaldéhyde et 5 gouttes d'acide acétique dans 15 ml de THF, on ajoute, par portions, 0,5 g de triacétoxyborohydrure de sodium et laisse une nuit sous agitation à TA. On ajoute ensuite 20 ml de MeOH et chauffe à 60°C pendant 2 heures. On concentre sous vide, reprend le résidu par une solution de NaOH à 30 %, extrait au DCM, sèche la phase organique sur Na₂SO₄ et évapore le solvant sous vide. On chromatographie le résidu sur gel de silice en éluant par le mélange DCM/MeOH (94/6 ; v/v). On obtient 0,271 g du produit attendu après cristallisation dans le mélange éther iso/DCM/éther, F = 104-105°C.
Spectre de masse : MH⁺ = 556,3.

### EXEMPLE 2 : Composé n° 2

Oxalate de N-méthyl-1-(1-méthyl-1*H-*imidazol-2-yl)-N-[[1-[(4-pyrazin-2-yl pipérazin-1-yl)acétyl]-4-[3-trifluorométhyl)phényl]pipéridin-4-yl]méthyl] méthanamine, dihydrate.

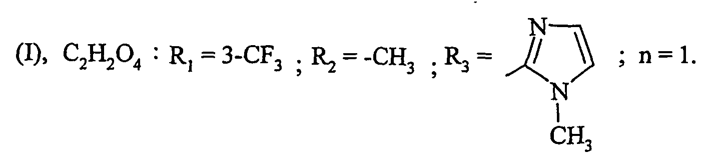

A un mélange de 0,5 g du composé obtenu à la Préparation 4.2, 0,115 g de 1-méthyl-1*H-*imidazole-2-carbaldéhyde et 3 gouttes d'acide acétique dans 10 ml de THF, on ajoute, par portions, 0,445 g de triacétoxyborohydrure de sodium et laisse une nuit sous agitation à TA. On concentre sous vide, reprend le résidu dans 10 ml de MeOH et chauffe à 70°C pendant 30 minutes. On concentre sous vide, reprend le résidu par une solution de NaOH à 30 %, extrait au DCM, sèche la phase organique sur Na₂SO₄ et évapore le solvant sous vide. On chromatographie le résidu sur gel de silice H en éluant par le mélange DCM/MeOH de (100/1 ; v/v) à (100/5 ; v/v). On reprend le produit obtenu dans l'éther, ajoute 0,082 g d'acide oxalique, laisse sous agitation et essore le précipité formé. On obtient 0,23 g du produit attendu, F = 80-100°C.
Spectre de masse : MH⁺ = 571,6.

Le tableau qui suit illustre les structures chimiques et les propriétés physiques de quelques exemples de composés selon l'invention. Dans ce tableau : - dans la colonne "sel", "-" représente un composé sous forme de base libre, alors que "HCl" représente un composé sous forme de chlorhydrate et "C₂H₂O₄" représente un composé sous forme d'oxalate.

**TABLEAU I**

| | | | | | | |
|---|---|---|---|---|---|---|
| 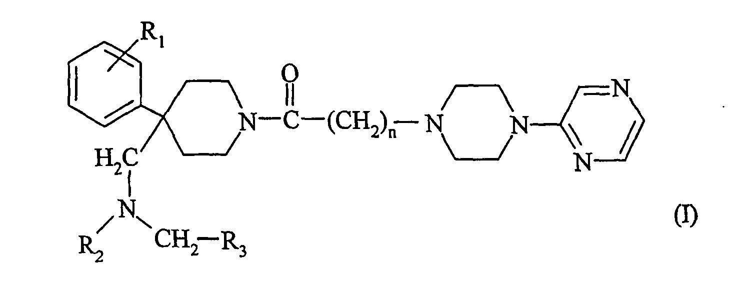 | | | | | | |

| Composés N° | R₁ | R₂ | R₃ | n | Sel | F°C ; Solvant de cristallisation MH⁺ |
|---|---|---|---|---|---|---|
| 1 | 3-CF₃ | H | 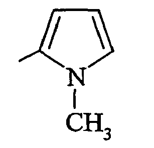 | 1 | - | 104-105 ; éther iso/ DCM/ether ; 556,3 |
| 2 | 3-CF₃ | -CH₃ | 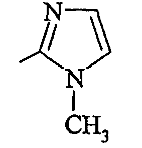 | | C₂H₂O₄ | 80-100; éther ; 571,6 |
| 3 | 3-CF₃ | -CH₃ | 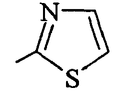 | 1 | 3HCl | - éther; 574,3 |
| (a) | | | | | | |
| 4 | 3-CF₃ | H | 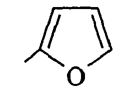 | 1 | - | 133-134 MeOH/éther ; 543,3 |
| (b) | | | | | | |
| 5 | 3-CF₃ | H | 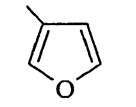 | 1 | - | 147 ; éther/DCM ; 543,3 |
| (b) | | | | | | |
| 6 | 3-CF₃ | H | 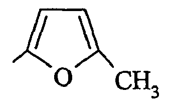 | 1 | - | 110-113 éther iso/DCM ; 557,5 |
| (b) | | | | | | |
| 7 | 3-CF₃ | -CH₃ | 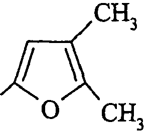 | 1 | 3HCl | - ether ; 585,4 |
| (a) | | | | | | |
| 8 | 3-CF₃ | -CH₃ | 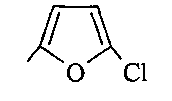 | 1 | - | 100-102 ; éther iso/DCM ; 591,4 |
| (a) | | | | | | |
| 9 | 3-CF₃ | H | 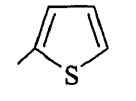 | 1 | - | 140-141 ; DCM/éther iso 559,3 |
| (b) | | | | | | |
| 10 | 3-CF₃ | H | 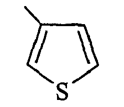 | 1 | - | 125-126 ; éther iso/DCM ; 559,3 |
| (b) | | | | | | |
| 11 | 3-CF₃ | H | 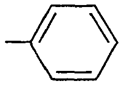 | 1 | - | 120 ; - 553,5 |
| (b) | | | | | | |
| 12 | 3-CF₃ | H | 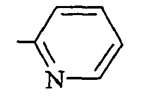 | 1 | - | 104-105; éther iso ; 554,4 |
| (b) | | | | | | |
| 13 | 3-CF₃ | -CH₃ | 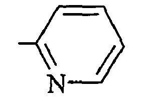 | 1 | - | 110-111; éther iso/DCM ; 568,5 |
| (a) | | | | | | |
| 14 | 3-CF₃ | -CH₃ | 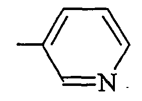 | 1 | 4HCl | - ether ; 568,4 |
| (a) | | | | | | |
| 15 | 3-CF₃ | -CH₃ | 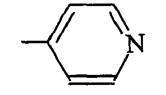 | 1 | 4HCl | - ether ; 568,4 |
| (a) | | | | | | |
| 16 | 3-CF₃ | -CH₃ | 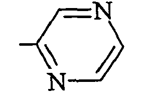 | 1 | 4HCl | - ether ; 569,4 |
| (a) | | | | | | |
| 17 | 3-CF₃ | H | 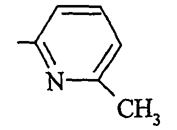 | 1 | - | 118-123 ; DCM/éther iso ; 568,2 |
| (b) | | | | | | |
| 18 | 3-CF₃ | H | 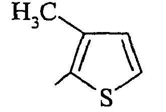 | 1 | 3HCl | - ether ; 573,2 |
| (b) | | | | | | |
| 19 | 3-CF₃ | -CH₃ | 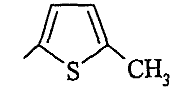 | 1 | HCl | - ether ; 587,2 |
| (a) | | | | | | |
| 20 | 3-CF₃ | -CH₃ | 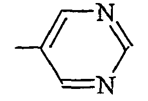 | 1 | - | 161 ; DCM/éther iso ; 569,2 |
| (a) | | | | | | |
| 21 | 3-CF₃ | -CH₃ | 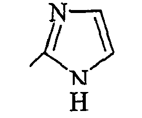 | 1 | - | 101-108 ; éther iso/DCM ; 557,2 |
| (a) | | | | | | |
| 22 | 3-CF₃ | -CH₃ | 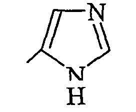 | 1 | 4HCl | - éther ; 557,2 |
| (a) | | | | | | |
| 23 | 3-CF₃ | -CH₃ | 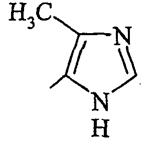 | 1 | - | 150-152 éther iso/DCM ; 571,2 |
| (a) | | | | | | |

| | | | | | | |
|---|---|---|---|---|---|---|
| (a) Composé préparé selon le mode opératoire décrit à l'Exemple 2 à partir du composé de la Préparation 4.2 et du composé de formule (III) correspondant. b) Composé préparé selon le mode opératoire décrit à l'Exemple 1 à partir du composé de la Préparation 4.1 et du composé de formule (III) correspondant. | | | | | | |

Les composés selon l'invention ont fait l'objet d'études biochimiques.

### Culture cellulaire :

La souche SH-SY-5Y (neuroblastome humain) est cultivée classiquement dans un milieu de culture DMEM (de l'anglais Dulbecco's Modified Eagle's Medium)(Gibco BRL, France) contenant du SVF (5%) (serum de veau foetal) (Boehringer Mannheim, Germany), du pyruvate de sodium (1 mM), de l'anti-PPLO( 5 ml) (agent anti mycoplasme : Tylocine^{®} préparée dans une solution saline normale, 6000 µg/ml), de la gentamycine (0.1mg/ml) et de la glutamine (4 mM) dans des flacons de culture recouvert de collagène (Becton Dickinson, France).

La souche mère SK-N-BE (neuroblastome humain) et le clone Bep 75 exprimant le récepteur p75^{NTR} humain (SK-N-BE Bep 75) sont cultivés classiquement dans un milieu de culture DMEM contenant du SVF (5%), du pyruvate de sodium (1 mM), de l'anti-PPLO( 5 ml) , de la gentamycine (0.1mg/ml) et de la glutamine (4 mM).

### Etude de la liaison du ¹²⁵I NGF au récepteur P75^{NTR}

L'étude de la liaison du ¹²⁵I NGF (le facteur de croissance neuronale radio marqué à l'iode-125) est réalisée sur une suspension cellulaire des deux souches SH-SY-5Y et SK-N-BE Bep 75 en accord avec la méthode décrite par Weskamp (Neuron, 1991, 6, 649-663). La liaison non spécifique est déterminée par la mesure de la liaison totale après une heure de pré-incubation avec les cellules à 37°C en présence de NGF non-radio marqué (1 µM). La liaison spécifique est calculée par différence entre la mesure de la liaison totale et la mesure de liaison non-spécifique. Les expériences de compétition sont réalisées en utilisant une concentration en ¹²⁵I NGF de 0.3 nM. Les concentrations inhibitrices de 50 % (CI₅₀) de la fixation de ¹²⁵I NGF au récepteur p75^{NTR} des composés selon l'invention sont faibles et varient de 10⁻⁶ à 10⁻¹¹M.

### Mesure de l'apoptose :

Les cellules (souches de neuroblastomes humains SH-SY-5Y et SK-N-BE Bep 75) sont installées dans des boîtes de Pétri de 35 mm de diamètre (Biocoat collagen I, (10⁵ cellules/puits) dans un milieu de culture DMEM contenant 5 % de SVF durant 24H. Le milieu de culture est ensuite éliminé, les cellules sont rincées avec du PBS (de l'anglais Dulbecco's Phosphate buffered saline) et soit du milieu frais contenant 5% de SVF soit du milieu contenant du NGF à la concentration de 10 ng/ml est ajouté en présence ou non des composés selon l'invention. Les taux d'apoptose sont mesurés 48 heures après les traitements dans le cas de la souche SH-SY-5Y et 24 heures après dans le cas de la souche SK-N-BE Bep 75 par quantification des histones cytoplasmiques associés aux fragments d'ADN (cell death detection ELISA, Boehringer Mannheim, Germany). Les taux d'apoptose sont exprimés en quantité d'oligonucléosomes/105 cellules ± DS. Chaque valeur correspond à la moyenne de 9 points expérimentaux répartis dans 3 expériences indépendantes. Les composés de formule (I) présentent une activité inhibitrice de l'apoptose induite par le NGF avec des CI₅₀ qui varient de 10⁻⁶ à 10⁻¹¹M_{.}

Ainsi la fixation des composés selon l'invention au récepteur p75^{NTR} se traduit d'une part au niveau biochimique par l'inhibition de la dimérisation du récepteur induit par les neurotrophines et d'autre part au niveau cellulaire par l'inhibition de l'effet proapoptotique médié par le récepteur p75^{NTR}.

Les composés selon l'invention peuvent donc être utilisés pour la préparation de médicaments, en particulier de médicaments destinés à prévenir ou à traiter toute pathologie où le récepteur p75^{NTR} est impliqué.

Ainsi, selon un autre de ses aspects, l'invention a pour objet des médicaments qui comprennent un composé de formule (I), ou un sel d'addition de ce dernier à un acide pharmaceutiquement acceptable, ou encore un solvat ou un hydrate du composé de formule (I).

Ainsi, les composés selon l'invention peuvent être utilisés, chez l'homme ou chez l'animal, dans le traitement ou la prévention de différentes affections p75^{NTR} dépendantes telles que les maladies neurodégénératives centrales et périphériques comme la démence sénile, l'épilepsie, la maladie d'Alzheimer, la maladie de Parkinson, la chorée d'Huntington, le syndrôme de Down, les maladies à prion, l'amnésie, la schizophrénie ; la sclérose latérale amyotrophique, la sclérose en plaques ; les affections cardiovasculaires comme les dommages cardiaques post-ischémiques, les cardiomyopathies, l'infarctus du myocarde, l'insuffisance cardiaque, l'ischémie cardiaque, l'infarctus cérébral ; les neuropathies périphériques (d'origine diabétique, traumatisme ou iatrogène) ; les dommages au nerf optique et à la rétine ; les traumatismes de la moëlle épinière et les traumatismes crâniens ; l'athérosclérose ; les sténoses ; la cicatrisation ; l'alopécie.

Les composés selon l'invention peuvent également être utilisés dans le traitement des cancers comme celui du poumon, de la thyroïde, du pancréas, de la prostate, de l'intestin grêle et du colon, du sein, dans le traitement des tumeurs, des métastases et des leucémies.

Les composés selon l'invention peuvent aussi être utilisés dans le traitement des douleurs chroniques neuropathiques et inflammatoires et dans le traitement des maladies auto-immunes comme la polyarthrite rhumatoïde.

Les composés selon l'invention peuvent également être utilisés dans le traitement des fractures osseuses, dans le traitement ou la prévention des maladies osseuses comme l'ostéoporose.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un solvat ou hydrate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | | |
|---|---|---|
| Composé selon l'invention | : | 50,0 mg |
| Mannitol | : | 223,75 mg |
| Croscarmellose sodique | : | 6,0 mg |
| Amidon de maïs | : | 15,0 mg |
| Hydroxypropyl-méthylcellulose | : | 2,25 mg |
| Stéarate de magnésium | : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I) : 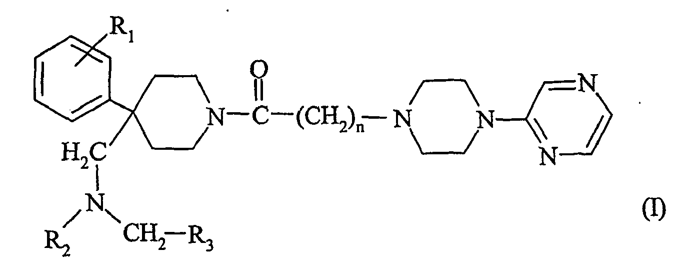 dans laquelle :
- n est 1 ou 2 ;
- R₁ représente un radical trifluorométhyle ;
- R₂ représente un atome d'hydrogène ou un (C₁-C₃)alkyle ;
- R₃ représente un groupe aromatique choisi parmi : 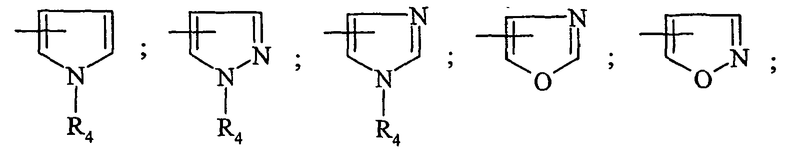 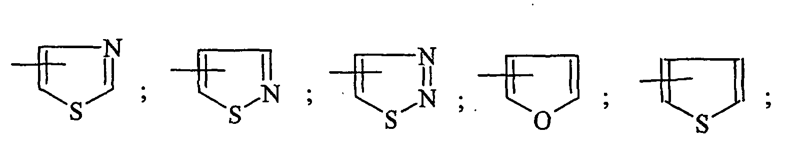 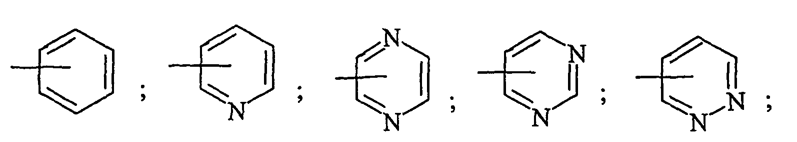 lesdits groupes aromatiques étant non substitués, mono- ou disubstitués par un substituant choisi indépendamment parmi un atome d'halogène, un (C₁-C₃)alkyle ou un (C₁-C₃)alcoxy ;
- R₄ représente un atome d'hydrogène ou un (C₁-C₃)alkyle ;
à l'état de base ou de sel d'addition à un acide, ainsi qu'à l'état d'hydrate ou de solvat.

2. Composé de formule (I) selon la revendication 1, **caractérisé en ce que** :
- n est 1 ;
à l'état de base ou de sel d'addition à un acide, ainsi qu'à l'état d'hydrate ou de solvat.

3. Composé de formule (I) selon la revendication 1, **caractérisé en ce que** :
- R₁ est en position -3- du phényle et représente un radical trifluorométhyle ;
à l'état de base ou de sel d'addition à un acide, ainsi qu'à l'état d'hydrate ou de solvat.

4. Composé de formule (I) selon la revendication 1, **caractérisé en ce que** :
- R₂ représente un atome d'hydrogène ou un radical méthyle ;
à l'état de base ou de sel d'addition à un acide, ainsi qu'à l'état d'hydrate ou de solvat.

5. Composé de formule (I) selon la revendication 1, **caractérisé en ce que** :
- R₃ représente un radical 1-méthyl-1*H*-pyrrol-2-yle ; 1-méthyl-1*H-*imidazol-2-yle ; 1,3-thiazol-2-yle ; 2-furyle ; 3-furyle ; 5-méthyl-2-furyle ; 4,5-diméthyl-2-furyle ; 5-chloro-2-furyle ; 2-thiényle ; 3-thiényle ; phényle ; pyridin-2-yle ; pyridin-3-yle ; pyridin-4-yle ; pyrazin-2-yle ; 6-méthylpyridin-2-yle ; 3-méthyl-2-thiényle ; 5-méthyl-2-thiényle ; pyrimidin-5-yle ; 1*H-*imidazol-2-yle ; 1*H-*imidazol-5-yle ; 4-méthyl-1*H-*imidazol-5-yle.
à l'état de base ou de sel d'addition à un acide, ainsi qu'à l'état d'hydrate ou de solvat.

6. Composé de formule (I) selon la revendication 1, **caractérisé en ce que** :
- n est 1 ;
- R₁ est en position -3- du phényle et représente un radical trifluorométhyle ;
- R₂ représente un atome d'hydrogène ou un radical méthyle ;
- R₃ représente un radical 1-méthyl-1*H-*pyrrol-2-yle ; 1-méthyl-1*H-*imidazol-2-yle ; 1,3-thiazol-2-yle ; 2-furyle ; 3-furyle ; 5-méthyl-2-furyle ; 4,5-diméthyl-2-furyle ; 5-chloro-2-furyle ; 2-thiényle ; 3-thiényle ; phényle ; pyridin-2-yle ; pyridin-3-yle ; pyridin-4-yle ; pyrazin-2-yle ; 6-méthylpyridin-2-yle ; 3-méthyl-2-thiényle ; 5-méthyl-2-thiényle ; pyrimidin-5-yle ; 1*H-*imidazol-2-yle ; 1*H-*imidazol-5-yle ; 4-méthyl-1*H-*imidazol-5-yle.
à l'état de base ou de sel d'addition à un acide, ainsi qu'à l'état d'hydrate ou de solvat.

7. Procédé de préparation des composés de formule (I) selon la revendication 1, **caractérisé en ce que** :
on fait réagir un composé de formule : 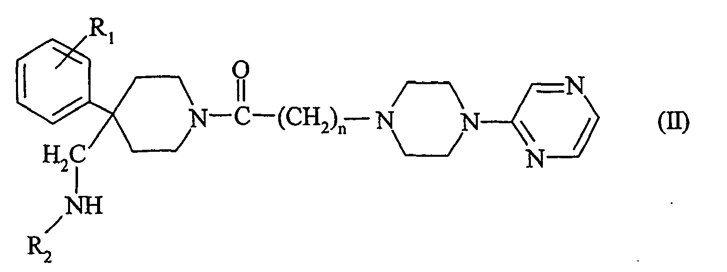
dans laquelle n, R₁ et R₂ sont tels que définis pour un composé de formule (I) dans la revendication 1, avec un composé de formule : 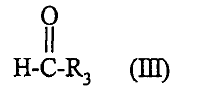 dans laquelle R₃ est tel que défini pour un composé de formule (I) dans la revendication 1, en présence d'un acide, dans un solvant, puis on réduit le sel d'iminium formé intermédiairement au moyen d'un agent réducteur.

8. Médicament, **caractérisé en ce qu'**il comprend un composé de formule (I) selon l'une quelconque des revendications 1 à 6, ou un sel d'addition de ce composé à un acide pharmaceutiquement acceptable, ou encore un hydrate ou un solvat du composé de formule (I).

9. Composition pharmaceutique, **caractérisé en ce qu'**elle comprend un composé de formule (I) selon l'une quelconque des revendications 1 à 6, ou un sel pharmaceutiquement acceptable, un hydrate ou un solvat de ce composé, ainsi qu'au moins un excipient pharmaceutiquement acceptable.

10. Utilisation d'un composé de formule (I) selon l'une quelconque des revendications 1 à 6 pour la préparation d'un médicament destiné à la prévention ou au traitement des maladies neurodégénératives centrales ou périphériques ; de la sclérose latérale amyotrophique, de la sclérose en plaques ; des affections cardiovasculaires ; des neuropathies périphériques ; des dommages au nerf optique et à la rétine ; des traumatismes de la moëlle épinière et des traumatismes crâniens ; de l'athérosclérose ; des sténoses ; de la cicatrisation ; de l'alopécie ; des cancers ; des tumeurs ; des métastases ; des leucémies ; des douleurs chroniques neuropathiques et inflammatoires ; des maladies auto-immunes ; des fractures osseuses ; des maladies osseuses.

## Claims

1. Compound of the formula (I); 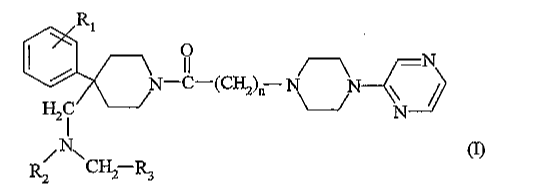 in which:
- n is 1 or 2;
- R₁ represents a trifluoromethyl radical;
- R₂ represents a hydrogen atom or a (C₁-C₃)alkyl;
- R₃ represents an aromatic group selected from: 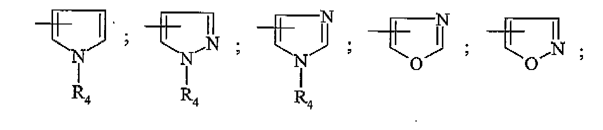 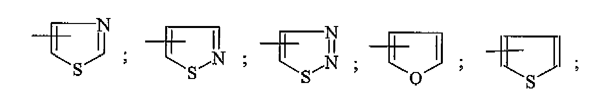 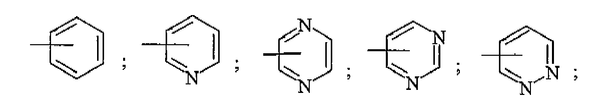 the said aromatic groups being unsubstituted or being mono- or disubstituted by a substituent selected independently from a halogen atom; a (C₁-C₃) alkyl or a (C₁-C₃)alkoxy;
- R₄ represents a hydrogen atom or a (C₁-C₃)alkyl;
in the form of a base or an addition salt with an acid, or in the form of a hydrate or solvate.

2. compound of formula (I) according to Claim 1, **characterized in that**:
- n is 1;
in the form of a base or an addition salt with an acid, or in the form of a hydrate or solvate.

3. Compound of formula (I) according to Claim 1, **characterized in that**:
- R₁ is in position 3 of the phenyl and represents a trifluoromethyl radical;
in the form of a base or an addition salt with an acid, or in the form of a hydrate or solvate.

4. Compound of formula (I) according to Claim 1, **characterized in that**:
- R₂ represents a hydrogen atom or a methyl radical;
in the form of a base or an addition salt with an acid, or in the form of a hydrate or solvate.

5. Compound of formula (I) according to Claim 1, **characterized in that**:
- R₃ represents a 1-methyl-1*H-*pyrrol-2-yl; 1-methyl-1*H-*imidazol-2-yl; 1,3-thiazol-2-yl; 2-furyl; 3-furyl; 5-methyl-2-furyl; 4,5-dimethyl-2-furyl; 5-chloro-2-furyl; 2-thienyl; 3-thienyl; phenyl; pyridin-2-yl; pyridin-3-yl; pyridin-4-yl; pyrazin-2-yl; 6-methylpyridin-2-yl; 3-methyl-2-thienyl; 5-methyl-2-thienyl; pyrimidin-5-yl; 1*H-*imidazol-2-yl; 1*H-*imidazol-5-yl; or 4-methyl-1*H-*imidazol-5-yl radical;
in the form of a base or an addition salt with an acid, or in the form of a hydrate or solvate.

6. Compound of formula (I) according to Claim 1, **characterized in that**;
- n is 1;
- R₁ is in position 3 of the phenyl and represents a trifluoromethyl radical;
- R₂ represents a hydrogen atom or a methyl radical;
- R₃ represents a 1-methyl-1*H-*pyrrol-2-yl; 1-methyl-1*H-*imidazol-2-yl; 1,3-thiazol-2-yl; 2-furyl; 3-furyl; 5-methyl-2-furyl; 4,5-dimethyl-2-furyl; 5-chloro-2-furyl; 2-thienyl; 3-thienyl; phenyl; pyridin-2-yl; pyridin-3-yl; pyridin-4-yl; pyrazin-2-yl; 6-methylpyridin-2-yl; 3-methyl-2-thienyl; 5-methyl-2-thienyl; pyrimidin-5-yl; 1*H-*imidazol-2-yl; 1*H-*imidazol-5-yl; or 4-methyl-1*H-*imidazol-5-yl radical;
in the form of a base or an addition salt with an acid, or in the form of a hydrate or solvate,

7. Process for preparing compounds of formula (I) according to Claim 1, **characterized in that**:
a compound of formula 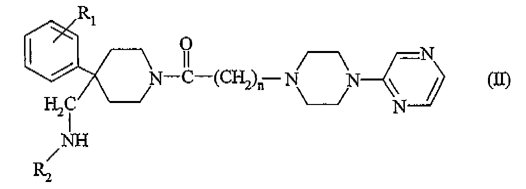
in which n, R₁ and R₂ are as defined for a compound of formula (I) in Claim 1, is reacted with a compound of formula 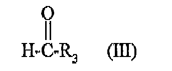
in which R₃ is as defined for a compound of formula (I) in Claim 1, in the presence of an acid and in a solvent and then the iminium salt intermediately formed is reduced by means of a reducing agent.

8. Medicament, **characterized in that** it comprises a compound of formula (I) according to any one of Claims 1 to 6, or an addition salt of this compound with a pharmaceutically acceptable acid, or else a hydrate or a solvate of the compound of formula (I).

9. Pharmaceutical composition, **characterized in that** it comprises a compound of formula (I) according to any one of Claims 1 to 6, or a pharmaceutically acceptable salt, a hydrate or a solvate of this compound, and at least one pharmaceutically acceptable excipient.

10. Use of a compound of formula (I) according to any one of Claims 1 to 6 for the preparation of a medicament intended for the prevention or treatment of central or peripheral neurodegenerative diseases; amyotrophic lateral sclerosis, multiple sclerosis; cardiovascular conditions; peripheral neuropathies; damage to the optic nerve and to the retina; spinal cord trauma and cranial trauma; atherosclerosis; stenoses; cicatrization; alopecia; cancers; tumours; metastases; leukaemias; chronic neuropathic and inflammatory pain; autoimmune diseases; bone fractures; bone diseases.

## Patentansprüche

1. Verbindung der Formel (I): 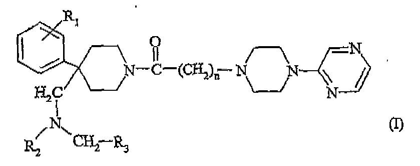 worin:
- n für 1 oder 2 steht;
- R₁ für einen Trifluormethylrest steht;
- R₂ für ein Wasserstoffatom oder ein (C₁-C₃)-Alkyl steht;
- R₂ für eine aromatische Gruppe steht, die unter: 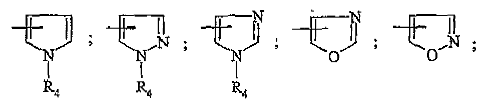 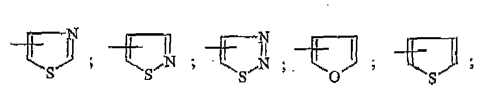 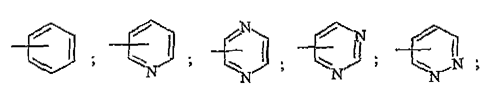 ausgewählt ist, wobei die aromatischen Gruppen gegebenenfalls ein- oder zweifach durch einen unabhängig voneinander unter einem Halogenatom, einem (C₁-C₃)-Alkyl oder einem (C₁-C₃)-Alkoxy ausgewählten Substituenten substituiert sind;
- R₄ für ein Wasserstoffatom oder ein (C₁-C₃)-Alkyl steht;
in Basen- oder Säureadditionssalzform sowie in Hydrat- oder Solvatform.

2. Verbindung der Formel (I) nach Anspruch 1, **dadurch gekennzeichnet, daß**:
- n für 1 steht;
in Basen- oder Säureadditionssalzform sowie in Hydrat- oder Solvatform.

3. Verbindung der Formel (I) nach Anspruch 1, **dadurch gekennzeichnet, daß**:
- R₁ in 3-Position des Phenyls steht und für einen Trifluormethylrest steht;
in Basen- oder Säureadditionssalzform sowie in Hydrat- oder Solvatform.

4. Verbindung der Formel (I) nach Anspruch 1, **dadurch gekennzeichnet, daß**:
- R₂ für ein Wasserstoffatom oder einen Methylrest steht;
in Basen- oder Säureadditionssalzform sowie in Hydrat- oder Solvatform.

5. Verbindung der Formel (I) nach Anspruch 1, **dadurch gekennzeichnet, daß**:
- R₃ für einen 1-Methyl-1*H-*pyrrol-2-yl-; 1-Methyl-1*H-*imidazol-2-yl-; 1,3-Thiazol-2-yl-; 2-Furyl-; 3-Furyl-; 5-Methyl-2-furyl-; 4,5-Dimethyl-2-furyl-; 5-Chlor-2-furyl-; 2-Thienyl-; 3-Thienyl-; Phenyl-; Pyridin-2-yl-; Pyridin-3-yl-; Pyridin-4-yl-; Pyrazin-2-yl-; 6-Methylpyridin-2-yl-; 3-Methyl-2-thienyl-; 5-Methyl-2-thienyl-; Pyrimidin-5-yl-; 1*H-*Imidazol-2-yl-; 1*H-*Imidazol-5-yl- oder 4-Methyl-1*H-*imidazol-5-ylrest steht;
in Basen- oder Säureadditionssalzform sowie in Hydrat- oder Solvatform.

6. Verbindung der Formel (I) nach Anspruch 1, **dadurch gekennzeichnet, daß**:
- n für 1 steht;
- R₁ in 3-Position des Phenyls steht und für einen Trifluormethylrest steht;
- R₂ für ein Wasserstoffatom oder einen Methylrest steht;
- R₃ für einen 1-Methyl-1*H-*pyrrol-2-yl-; 1-Methyl-1*H-*imidazol-2-y1-; 1,3-Thiazol-2-yl-; 2-Furyl-; 3-Furyl-; 5-Methyl-2-furyl-; 4,5-Dimethyl-2-furyl-; 5-Chlor-2-furyl-; 2-Thienyl-; 3-Thienyl-; Phenyl-; Pyridin-2-yl-; Pyridin-3-yl-; Pyridin-4-yl-; Pyrazin-2-yl-; 6-Methylpyridin-2-yl-; 3-Methyl-2-thienyl-; 5-Methyl-2-thienyl-; Pyrimidin-5-yl-; 1*H-*Imidazol-2-yl-; 1*H-*Imidazol-5-yl- oder 4-Methyl-1*H-*imidazol-5-ylrest steht;
in Basen- oder Säureadditionssalzform sowie in Hydrat- oder Solvatform.

7. Verfahren zur Herstellung von Verbindungen der Formel (I) nach Anspruch 1, **dadurch gekennzeichnet, daß** man:
eine Verbindung der Formel: 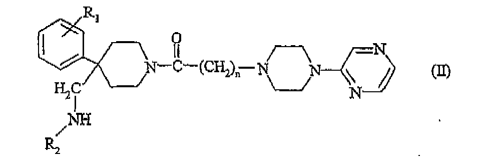
worin n, R₁ und R₂ die für eine Verbindung der Formel (I) in Anspruch 1 angegebene Bedeutung besitzen, in Gegenwart einer Säure in einem Lösungsmittel mit einer Verbindung der Formel: 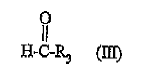
worin R₃ die für eine Verbindung der Formel (I) in Anspruch 1 angegebene Bedeutung besitzt, umsetzt und dann das intermediär gebildete Iminiumsalz mit Hilfe eines Reduktionsmittels reduziert.

8. Arzneimittel, **dadurch gekennzeichnet, daß** es eine Verbindung der Formel (I) nach einem der Ansprüche 1 bis 6 oder ein Additionssalz dieser Verbindung mit einer pharmazeutisch unbedenklichen Säure oder auch ein Hydrat oder ein Solvat der Verbindung der Formel (I) enthält.

9. Pharmazeutische Zusammensetzung, **dadurch gekennzeichnet, daß** sie eine Verbindung der Formel (I) nach einem der Ansprüche 1 bis 6 oder ein pharmazeutisch unbedenkliches Salz, ein Hydrat oder ein Solvat dieser Verbindung sowie mindestens einen pharmazeutisch unbedenklichen Hilfsstoff enthält.

10. Verwendung einer Verbindung der Formel (I) nach einem der Ansprüche 1 bis 6 zur Herstellung eines Arzneimittels zur Prävention oder Behandlung von zentralen oder peripheren neurodegenerativen Erkrankungen; amyotropher Lateralsklerose, multipler Sklerose; Herz-Kreislauf-Leiden; peripheren Neuropathien; Schäden des Sehnervs und der Retina; Rückenmarks- und Schädeltraumata; Atherosklerose; Stenosen; Vernarbung; Alopecia; Krebserkrankungen; Tumoren; Metastasen; Leukämien; chronischen neuropathischen und entzündlichen Schmerzen; Autoimmunerkrankungen; Knochenbrüchen und Knochenerkrankungen.
